# EUROPÄISCHE PATENTSCHRIFT

(11) **EP 0 269 801 B1**
(45) Veröffentlichungstag und Bekanntmachung des Hinweises auf die Patenterteilung: **29.12.1993**
(21) Anmeldenummer: 87113695.8
(22) Anmeldetag: 18.09.1987
(51) Int. Cl.: A61B 6/04, A61B 17/22

(54) **Rechnerkontrollierte Patientenpositionierung**
Positioning of a patient by a controlled computer
Positionnement de malade par un ordinateur commandé

(30) Priorität: 14.11.1986 DE 3638953
(43) Veröffentlichungstag der Anmeldung: 08.06.1988
(73) Patentinhaber: DORNIER MEDIZINTECHNIK GMBH, D-82101 Germering (DE)
(72) Erfinder: Wess, Othmar, Dr., D-8000 München 81 (DE); Novak, Pavel, Dr., D-8000 München 2 (DE); Mechnich, Klaus, D-8031 Wessling (DE); Schultheiss, Reiner, D-8088 Eching (DE)
(74) Vertreter: Landsmann, Ralf, Dipl.-Ing.

(56) Entgegenhaltungen:
- EP-A- 0 139 941
- EP-A- 0 168 559
- EP-A- 0 225 104
- DE-A- 3 122 056
- DE-A- 3 220 751

## Beschreibung

Die Erfindung betrifft eine Ortungs- und Positionier. Vorrichtung nach dem Oberbegriff des Anspruchs 1. Sie dient zur automatischen Positionierung eines sich auf einer Liege befindlichen Patienten, der in eine exakt vorherbestimmte Körperlage zu versetzen ist.

Aus der DE-PS 23 51 247 ist eine Vorrichtung mit Fokussierkammer zur Zerkleinerung von Konkrementen, die sich im Körper eines Lebewesens befinden, bekannt, wobei die Fokussierkammer ein Teil eines Rotationsellipsoiden ist, in dessen einem Brennpunkt Stosswellen durch Funkenentladung erzeugbar sind. Die Kammer ist dabei mit einer Flüssigkeit gefüllt und das zu zerkleinernde Konkrement befindet sich im zweiten Brennpunkt des Ellipsoiden. Mittels einer Funkenstrecke wird durch elektrische Unterwasser-Funkenentladung die in einem Kondensator gespeicherte Energie in mechanische Stosswellenenergie umgewandelt. Durch Zündung dieser Funkenentladung in einem Brennpunkt des Rotationsellipsoiden lassen sich nahezu punktförmig im zweiten Brennpunkt Stosswellen hoher Amplitude (größer 1 kbar mit geringen Impulslängen kleiner 1 µsec) erzeugen, die in Körpern von Lebewesen befindliche Konkremente zu einem feinen Gries zerkleinern, der auf natürliche Weise aus dem Körper herausgeschwemmt wird.

Der Nierenstein wird dabei beispielsweise mittels zweier Röntgenabbildungssysteme in seiner Größe und seiner Lage im Körper des Patienten lokalisiert.

Aus der EP 168 559 A1 ist eine gattungsgemäße Ortungs- und Positioniereinrichtung bekannt, bei der durch die Kombination von Röntgen- und Ultraschall-Ortungssystemen die Lage des Nierensteins ständig beobachtet und exakt festgestellt werden kann und bei der der Behandlungserfolg ebenfalls durch die hohe Auflösung des Röntgensystems sichtbar ist. Hierbei wird von einer automatisierten Verschiebung gesprochen, die jedoch nur innerhalb der Ultraschall-Schnittebene geschehen kann. Dann ist ebenfalls ausser der Röntgenortungseinrichtung noch als zweite Ortungseinrichtung eine Ultraschallanlage erforderlich. Bei einer Verschiebung innerhalb der Ultraschallebene kann mit einem Lightpen der Steinort auf dem Bildschirm, der die Ultraschall-Schnittebene darstellt, angesteuert oder über Rechnertastatur eingegeben werden.

In der Münchner Medizinischen Wochenschrift, 125 (1983) Nr. 8 ist auf den Seiten 151 bis 155 eine Vorrichtung zur Zerkleinerung von Nierensteinen durch Stosswellen beschrieben, bei der das Konkrement zusammen mit dem Patient in den Fokusbereich gebündelter Stosswellen gebracht wird. Dies geschieht durch eine motormässig betriebene, vom Arzt aber von Hand gesteuerte Positioniereinrichtung. Den Steinort überwacht der Arzt dabei mittels zweier unabhängiger Röntgenortungssysteme.

Eine gleichwirkende Vorrichtung ist aus der DE 32 20 751 A1 bekannt. Sie enthält zwei unabhängige Röntgen- oder Ultraschall-Ortungssysteme und eine davon unabhängige, vom Arzt ebenfalls von Hand zu steuernde Positionierung. Als Positionierhilfen dienen Faden Kreuze, die die Sollage der Nierensteine markieren Aufgabe der Erfindung ist, das zu zertrümmernde Konkrement sicher zu erkennen, schnellstmöglich ohne Zuhilfenahme des Bedienungspersonals in den zweiten Fokus (F2) des Rotationsellipsoiden zu bringen, dabei die Belastung des Patienten und des Personals durch Röntgenstrahlung so gering wie möglich zu halten und die wichtige, luftblasenfreie Verbindung zwischen Wasserkissen und Patient nicht abreissen zu lassen.

Diese Aufgabe wird erfindungsgemäss gelöst durch die Vorrichtung nach Anspruch 1. Ausführungen der Erfindung sind Gegenstände von abhängigen Ansprüchen.

Der Patient befindet sich auf einer Liege, die in den Positioniertisch einsetzbar ist. Gleichzeitig ist die Liege auch in einem Fahrschemel einsetzbar, mit dem der Patient in den Behandlungsraum gefahren werden kann, ohne dass dort seine Lagerung noch einmal geändert werden müsste.

Mit einer Handbedienung, auf deren Bedientasten die Bewegungsmöglichkeiten der Positioniereinrichtung symbolisch dargestellt sind, ist die Übernahmeposition zur Umlagerung der Patientenliege vom Liegewagen (Fahrschemel) auf den Positioniertisch und umgekehrt ansteuerbar. Bei Erreichen der Position zeigen LED's auf der Handbedienung den momentanen Status an. Im weiteren umfasst der Tastenvorrat der Handbedienung die Bewegungsrichtungen entlang der
- x-Achse (fußwärts/kopfwärts)
- y-Achse (links/rechts)
- z-Achse (auf/ab)
- w-Achse (Kippung um die Liegenlängsachse zur Vermeidung von Verdeckungen des Konkrements durch Rippen oder Wirbelsäule)
- Röntgenschirmkoordinaten (horizontal/vertikal, wobei bei der Horizontalbewegung das Konkrement nur auf einem, dem jeweils eingeschalteten Monitor bewegt wird).

In der Behandlungposition liegt der Patient mit dem Rücken auf einem Wasserkissen. Zwischen Kissen und Patient befindet sich eine Ultraschall-Koppelgelschicht zur Vermeidung von Hohlräumen und Luftblasen zwischen den beiden Oberflächen. Dennoch vorhandene Luftblasen werden mit einem Wischer von der Innenseite des Kissen aus beseitigt. Ultraschall-Koppelgel ist bevorzugt einsetzbar, da es einfach zu beschaffen ist und keine negativen, stosswellenhemmenden Eigenschaften hat. Das Niveau des Wasserkissens ist ebenfalls bei Bedürfnis per Tastendruck absenkbar und anhebbar.

Um das zu zertrümmernde Konkrement auf den Röntgenbildschirmen sichtbar zu machen, wird der Positioniertisch über die Handsteuerung verfahren, bis das Konkrement auf beiden Schirmen sichtbar ist. Dabei wird das Niveau des Wasserkissens in Abhängigkeit von der Bewegung des Positioniertisches rechnergesteuert nachgeführt, so dass sichergestellt ist, dass der für die Stosswellenbehandlung erforderliche Kontakt zwischen Patient und Wasserkissen während der Positionierung nicht abreisst.

Die folgende Ortung des Konkrements wird rechnergesteuert vorgegeben. Die Ermittlung der Konkrementlage geschieht durch ein gekreuztes Röntgensystem. Damit erhält man zwei ebene Projektionen unter verschiedene, aber momentan fixen Winkeln. Die Ermittlung der Projektionskoordinaten in den Röntgenprojektionen erfolgt über das Videobild der Röntgenanlage. Zunächst erfolgt eine Röntgenaufnahme der momentanen Konkrementlage. Danach ist das Konkrement auf beiden Röntgenmonitoren sichtbar und das Lightpensystem wird aktiviert. Dabei liegt das Konkrement irgendwo im dargestellten Bereich. Zunächst wird der zweite Monitor abgeschaltet und der Benutzer aufgefordert, die Konkrementposition auf dem eingeschalteten ersten Monitor mit dem Lightpen einzugeben. Falls korrekte Werte, also Werte innerhalb vorgegebener Grenzen, eingelesen wurden, wird der erste Monitor abgeschaltet und der Benutzer aufgefordert, die Position auf dem zweiten Monitor einzugeben. Ebenfalls möglich wäre eine Version, bei der ursprünglich nicht beide Monitore eingeschaltet sind, sondern direkt nur der Monitor, von dem eingelesen wird, zunächst der erste Monitor, dann der zweite Monitor.
Lightpen besitzt in der Spitze einen Drucktaster, der betätigt wird, wenn der Lightpen den Bildschirm berührt. Dadurch wird die elektronische Positionsabfrage gestartet. Hierzu wird die Zeit ermittelt, die der Elektronenstrahl vom Videobildanfang bis zum angelegten Lightpen benötigt (Fernseh-Zeilenraster). Der Elektronenstrahl wird vom Lightpen mittels einer Photodiode mit angeschlossenem Schwellwert-Diskriminator erfasst.
Für die Ermittlung der Zeit können entweder ein oder zwei Zähler verwendet werden. Die zweite Lösung bietet grössere Genauigkeit, indem der eine Zähler die Zeilenzahl und der andere Zähler nur die Zeit vom Anfang der Zeile bis zum Lightpen erfasst. Eine weitere Genauigkeitssteigerung ist durch die Messung der gesamten Zeilenlänge, die dann zur Normierung der Teilzeit benutzt wird (Kompensation der Zeilenfrequenzschwankungen), möglich.
Die so ermittelten virtuellen Koordinaten (Zeilenzahl, Zeilen-Teilzeit) werden in reelle Koordinaten x, y mit Ursprung im Bildschirmmittelpunkt umgerechnet. Die Positionseingabe ist damit abgeschlossen.

Die Koordinaten-Eingabe, die hier mit Lichtgriffel beschrieben ist, kann grundsätzlich auch mit anderen Mitteln, wie Tracking-Ball, Joystick oder Mouse erfolgen. Hierzu muss in das Videobild beispielsweise ein Fadenkreuz oder ein Pfeil eingeblendet werden.

Anschliessend wird durch Drücken einer beliebigen Taste der Ansteuerung die automatische Positionierung eingeleitet. Dabei berechnet zunächst ein Leitrechner, der die Koordinaten des Lichtgriffels direkt abfragt, über eine mathematische Beziehung die räumliche Koordinate des Konkrementes (x, y, z). Bezugspunkt (oder Koordinatenursprung) ist der geometrische zweite Fokus (F2) des Rotationsellipsoids (x_{F2}, y_{F2}, z_{F2}). Der Leitrechner gibt danach den Achsrechnern (Indexer) Steuersignale, um das Konkrement aus (x, y, z) in den Fokus der Stosswelle (x_{F2}, y_{F2}, z_{F2}) zu befördern.

Vier der Eingangs bezeichneten Achsen werden dabei servomotorisch gesteuert, und zwar die x-, y-, z- und w-Achse. Jede Achse besitzt einen Indexer, der dem Leitrechner den jeweiligen Achsenstatus meldet, die Patientenbewegung über die Patientenpositioniereinrichtung kontrolliert, indem er die Koordinaten über Wegaufnehmer abfragt und die Bewegung stoppt, falls das Konkrement den zweiten Fokus F2 erreicht hat. Drei Befehlsausführungen sind möglich:
- Bewegung mit konstanter Geschwindigkeit
- Zielbewegung auf einen vorgegebenen Koordinatenpunkt
- Statusabfrage.

Die Bewegung der Patientenliege erfolgt geschwindigkeitsgeregelt mit einer maximalen Geschwindigkeit von ca. 10 mm/sec, wobei beim Anfahren und Abbremsen Beschleunigungs- und Bremsrampen gefahren werden können. Durch Kombination der verschiedenen Antriebe der Achsen können innerhalb der Bewegungsgrenzen Bewegungen auf beliebigen Bahnen bzw. isozentrische Bewegungen durchgeführt werden.

Die einzelnen Eingaben und Vorgänge können vom Benutzer auf einem Rechnermonitor beobachtet werden. Die momentan anstehende Eingabe ist dabei auf dem Bildschirm hervorgehoben, so dass der Benutzer jederzeit weiß, an welcher Stelle des Positioniervorganges er sich befindet. Der Rechner durchläuft automatisch die einzelnen Schritte der Positionierung, aber die einzelnen Betriebsmodi können innerhalb des Bedienermenues auf dem Bildschirm auch über Tastatur frei angewählt werden. Zusätzlich kann die Positionsschutzfunktion (Berührung des Ellipsoidrandes durch den Patienten) abgewählt werden, damit auch beleibte Patienten behandelt werden können. Ebenso werden auf dem Bildschirm im Bedienermenue die ermittelten Soll-Koordinaten, die aktuelle Patienten-Tisch-Koordinaten und das aktuelle Wasserkissenniveau angezeigt.

Um eine sichere Positionierung des Patienten zu gewährleisten, ist eine Reihe von Sicherheitsfunktionen in das System integriert:
a) Funktionsüberprüfung bei der Inbetriebnahme durch den Leitrechner, indem eine Bewegung durchgeführt wird und durch einen automatischen Ist-/Soll-Koordinatenvergleich Übereinstimmung geprüft wird. Bei nicht vorhandener Übereinstimmung wird die Fehlermeldung "Positioniersystem defekt" auf dem Bildschirm ausgegeben.
b) Sämtliche Bewegungen der Handpositionierung und automatischen Positionierung werden gemäss dem "Totmannprinzip" ausgelöst, wird also die betreffende Taste losgelassen, so stoppt die dazugehörige Bewegung automatisch.
c) Bewegungssoftware-Grenzen im Leitrechner und in den Achsrechnern, wobei bei kombinierter Bewegung in mehreren Achsrichtungen die Bewegung gestoppt wird, sobald in einer Koordinatenrichtung eine Software-Grenze erreicht wird.
d) Bewegungshardware-Grenzen durch montierte Endschalter an den Enden der jeweiligen Achsen. Über die Endschalter wird zum einen der jeweilige Achsrechner und zum anderen die dazugehörige Leistungsendstufe beeinflusst.
e) Die Stromversorgung der Leistungsendstufen wird vom Rechner nur während des Positionierens eingeschaltet.
f) Eine Bewegung wird nur dann ausgelöst, wenn vom Achsrechner an die Leistungsendstufe neben dem analogen Geschwindigkeitssteuersignal auch noch ein zusätzliches Enable-Signal gegeben wird.
g) Die Signalübertragung von den absoluten Lagegebern erfolgt störsicher mit einer Zwei-Draht-Übertragung.
h) Für die Eingabe der Konkrementlage mittels Lightpen sind eigene Fehlermeldungen vorgesehen:
   - "keine Positionseingabe"
      Diese Fehlermeldung erscheint auf dem Monitor, wenn innerhalb von 30 sec nach Aufforderung durch das Bedienermenue keine Eingabe durch den Lightpen vorgenommen wurde.
   - "Positionseingabe ungenau"
      Diese Fehlermeldung erscheint, wenn die eingelesenen Daten zu sehr streuen. Beim Antippen des Schirmes werden jeweils 10 Wertepaare eingelesen, die der Zähler des Lightpens ermittelt. Der Bereich zwischen 0 und dem maximal ermittelten Wert wird in x-Gruppen unterteilt, wobei x = Maximalwert/10 ist. Dann werden die ermittelten Werte in Gruppen eingeteilt, wobei jede Gruppe einen gewissen Zahlenbereich abdeckt. Aus der Gruppe, in der mehr als 5 Werte liegen, wird der Mittelwert und die Standardabweichung errechnet. Liegt die Standardabweichung bei größer 0,01, erscheint die Fehlermeldung.
   - "Positionseingabe inkorrekt"
      Diese Fehlermeldung erscheint, wenn die insgesamt eingegebenen Koordinaten nicht zu einem räumlichen Punkt führen, der maximal einer Kugel mit 10 mm Durchmesser entspricht.
   - "Position nicht erreichbar"
      Die maximale Bewegungsfreiheit auf jeder Achse beträgt ca. ± 10 cm. Liegt die jeweils errechnete Koordinate ausserhalb dieses Bewegungsbereiches, so erscheint die Fehlermeldung. Es ist auch möglich, direkt auf dem Bildschirm nach Erreichen eines Bewegungsrichtungsendes anzugeben, in welche Richtung der Patient umgelagert werden muss.
i) Meldung bei nicht Übereinstimmung der Soll/Ist-Koordinaten bei der automatischen Positionierung, beispielsweise dadurch hervorgerufen, dass die Auslösetaste zu früh losgelassen wurde.

Um die Sicherheit noch weiter zu erhöhen, wird nach Abschluß des Positioniervorganges eine Kontrollaufnahme durch das Röntgensystem durchgeführt.

Die vorgeschlagene Positioniereinrichtung verkürzt die Ortungs- und Positionierzeit durch einmaliges Orten und automatische rechnerkontrollierte Positionierung wesentlich. Die Positionierung wird weitgehendst bedienerunabhängig. Daraus resultiert eine Minimierung der Strahlenbelastung für Patient und Arzt/Personal und die Sicherheit für den Patient erhöht sich, da Fehlpositionierungen bei richtig erkanntem Stein ausgeschlossen sind. Der Patientendurchsatz lässt sich ebenfalls steigern. Da das Wasserkissen der Patientenbewegung in z-Richtung automatisch nachgeführt wird, bleibt die Ankopplung zwischen Patient und Kissenabschlußmembran ständig erhalten.

Ebenso kann die Eingabe der Konkrementposition auch mit einer Touch-Screen erfolgen. Eine Touch-Screen ist ein Bildschirmvorsatz mit einem Infrarotstrahl-Gitter oder einer speziellen druckempfindlichen Folie. Die Position kann dann einfach mit dem Finger auf der Screen angetippt werden.

Um den geforderten Sicherheitsaspekten und -vorschriften gerecht zu werden, ist es erforderlich, die Kopplung zwischen Röntgenanlage und Rechner bzw. Meßsystem so minimal wie möglich zu halten. Deshalb benötigt und benutzt die erfindungsgemässe Vorrichtung statt einer Komplettdigitalisierung des dargestellten Röntgenbildes nur die aus dem Röntgen-Videobild herausgefilterten Bildsynchron- und gegebenenfalls Zeilensynchronimpulse.

Die Erfindung wird anhand von Figuren näher erläutert.
- Figur 1: zeigt ein Prinzipschaltbild der Positioniereinrichtung;
- Figur 2: zeigt die Abbildung des Bedienermenues auf dem Monitor,
- Figur 3: zeigt eine schematische Darstellung des Lightpen-Meßsystems.

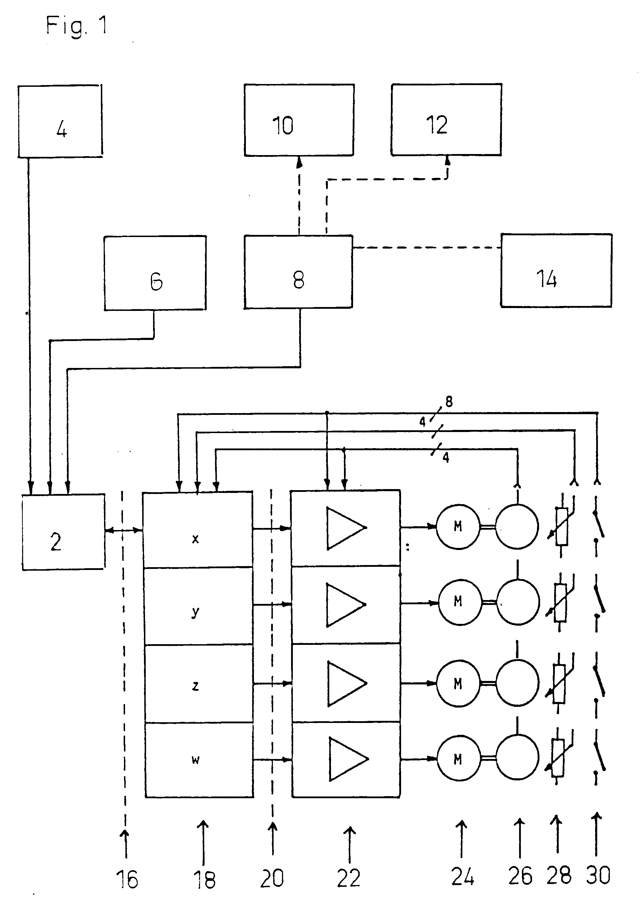

Figur 1 zeigt den zentralen Leitrechner 2, an den Bedienungsterminal 4, Handbedienung 6 und Lightpen-Meßsystem 8 angeschlossen sind. Das Lightpen-Meßsystem 8 steht mit den beiden Röntgenmonitoren 10 und 12 und dem damit gekoppelten Röntgensystem 14 in Verbindung. Über eine erste Schnittstelle 16 werden die Befehle des Leitrechners 2 an die vier Achsrechner (Indexer) 18 übermittelt, die für die Bewegungen in den vier Achsrichtungen x, y, z, w zuständig sind.

Über eine zweite Schnittstelle 20 ist jeder Achsrechner 18 mit je einer Leistungsendstufe 22 verbunden, die ihrerseits je einen Motor 24 je Achse mit angeflanschtem optischem Encoder 26 oder anderem Geber wie Tachogenerator oder Resolver steuert. Der optische Encoder 26 beeinflusst die Motorregelung sowohl in der Leistungsendstufe 22 als auch im Achsrechner 18. Zur Bestimmung der aktuellen Position auf den jeweiligen Achsen sind diese Achsen mit Absolutgebern 28 bestückt, die ihre Lageinformation zu den Achsrechnern senden, die sie an den Leitrechner 2 weitervermitteln. An den Enden jeder Achse sind mechanische Endschalter 30 befestigt, die neben den Software-Endschaltern dazu dienen, den Achsrechnern 18 und den Leistungsstufen 22 anzuzeigen, dass eine weitere Bewegung in dieser Richtung nicht möglich ist. Sie schalten die Leistungsstufe 22 sofort ab. Das Rechnersystem und die Röntgenanlage sind in dem Lightpen-Meßsystem 8 voneinander galvanisch getrennt.

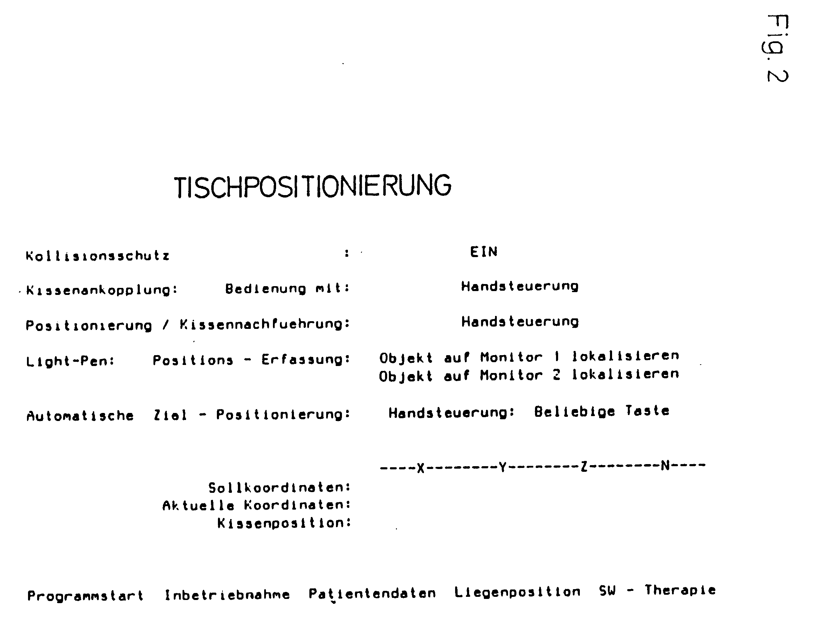

Figur 2 zeigt die Abbildung des Bedienermenues auf dem Monitor des Terminals 4 aus Figur 1. Das Menue wird vom Rechner automatisch zeilenweise durchgegangen und die aktuelle Angabe ist dabei hervorgehoben. Ebenso werden die Soll-Koordinaten der Konkrementlage und die sich während des Positioniervorganges ständig ändernden aktuellen Koordinaten und das Wasserkissenniveau angezeigt. Soll-Koordinaten und aktuelle Koordinaten müssen zum Zeitpunkt des Beschusses des Konkrements übereinstimmen. Über die Koordinaten ist auch ablesbar, ob und wo die Positioniereinrichtung ein Bewegungsende erreicht hat.

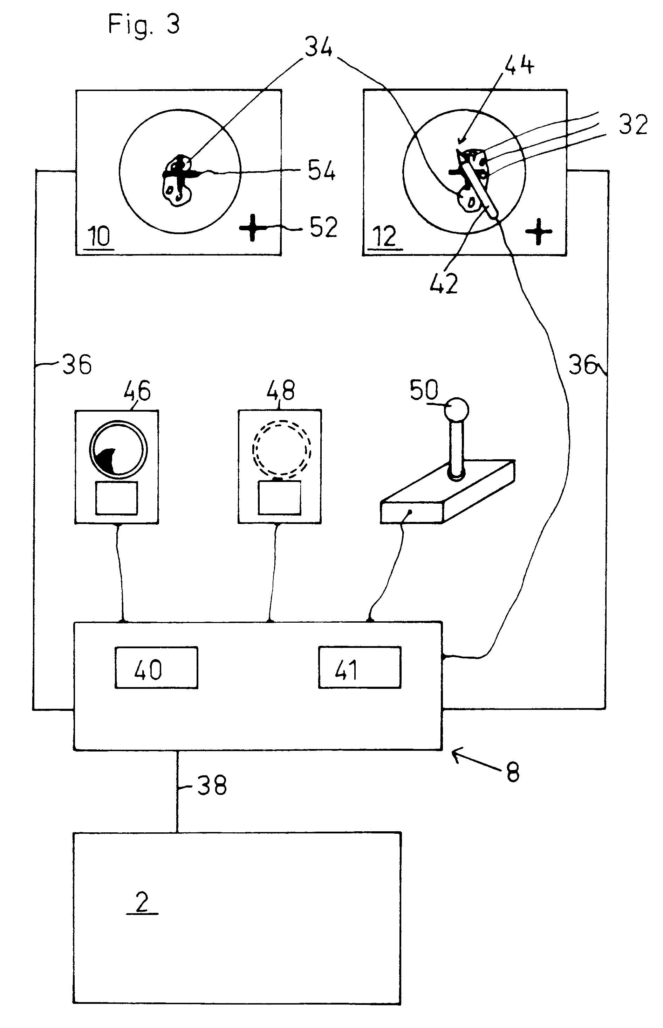

Figur 3 zeigt eine schematische Darstellung des Meßsystems 8 zur Aufnahme der Lagekoordinaten der zu behandelnden Konkremente 32. Auf den beiden Röntgenmonitoren 10 und 12 sind die Konkremente 32 in Nieren 34 abgebildet. Die Monitore 10 und 12 sind über Zuleitungen 36 mit dem Meßsystem 8 verbunden. Über eine weitere Leitung 38 sind Meßsystem 8 und Leitrechner 2 gekoppelt. Innerhalb des Meßsystem 8 sind die beiden Zähler 40 und 41 zur Bestimmung der Bildschirmkoordinanten gezeigt. Mit dem Meßsystem 8 verbunden ist der Lightpen 42, an dessen Spitze sich der Drucktaster 44 befindet. Alternativ zum Lightpen 42 sind Tracking-Ball 46, Mouse 48 und Joystick 50 aufgezeigt, ebenfalls in Verbindung mit dem Meßsystem 8. Bei den drei letztgenannten Lageeingabegeräten 46, 48 und 50 ist ein zusätzliches Fadenkreuz 52 zum mittigen Fadenkreuz 54 in jeden Bildschirm 10 und 12 eingeblendet.

## Patentansprüche

1. Ortungs- und Positionierungsvorrichtung in einer Einrichtung für die berührungsfreie Zerkleinerung von Konkrementen in Körpern von Lebewesen mit einem Wasserkissen, über das Stosswellen in den Körper eingeleitet werden, mit einer rechnergestützten Lagekoordinatenauswertung der Konkremente und mit einer rechnergesteuerten Positionierung einer Patientenliege, **gekennzeichnet durch**
- eine rechnergesteuerte Nachführung des Niveaus des Wasserkissens und
- die rechnergesteuerte Positionierung eines georteten Konkrements (**32**) in den zweiten Fokus eines Rotationsellipsoids auf Achsen in
x-Richtung (fußwärts/kopfwärts),
y-Richtung (links/rechts),
z-Richtung (auf/ab) und
w-Richtung (Kippung um die Längsachse).

2. Vorrichtung nach Anspruch 1, **dadurch gekennzeichnet**, daß mindestens ein, vorzugsweise zwei, Röntgen- oder Ultraschallortungssysteme (**10, 12, 14**) vorgesehen sind.

3. Vorrichtung nach wenigstens einem der vorherigen Ansprüche, **dadurch gekennzeichnet**, daß die Lagekoordinaten durch ein Bildverarbeitungssystem (**2, 8, 10, 12, 14**) aufgenommen sind.

4. Vorrichtung nach wenigstens einem der vorherigen Ansprüche, **dadurch gekennzeichnet**, daß das Bildverarbeitungssystem aus zwei Röntgenaufzeichnungsgeräten (**14**), deren Zentralstrahlen sich kreuzen, zwei damit gekoppelten und dazu gehörigen Monitoren (**10**, **12**), einem Lightpen-Meßsystem (**8**), das mit den Monitoren (**10, 12**) und den Röntgenaufzeichnungsgeräten (**14**) in Verbindung steht, und einem an dem Lightpen-Meßsystem (**8**) angeschlossenen Leitrechner (**2**) zusammengesetzt ist.

5. Vorrichtung nach einem der vorherigen Ansprüche, **dadurch gekennzeichnet**, daß jede Achse über einen separaten Achsrechner (**18**), eine Leistungsendstufe (**22**), einen Motor (**24**) mit angeflanschtem Geschwindigkeits- und Winkelgeber (**26**), einem absoluten Lagegeber (**28**) und einem mechanischen Endschalter (**30**) verfügt.

6. Vorrichtung nach einem der vorherigen Ansprüche, **dadurch gekennzeichnet**, daß alle Bewegungen der Positioniereinrichtung auch über eine Handbedienung (**6**) steuerbar sind.

7. Vorrichtung nach einem der vorherigen Ansprüche, **dadurch gekennzeichnet**, daß die Positioniereinrichtung sich nur solange bewegt, wie eine Auslösetaste gedrückt ist (Totmannprinzip).

8. Vorrichtung nach einem der vorherigen Ansprüche, **dadurch gekennzeichnet**, daß das Überschreiten der Bewegungsgrenzen sowohl durch Bewegungssoftware-Begrenzer als auch durch Bewegungshardware-Begrenzer (**30**) verhindert ist.

9. Vorrichtung nach einem der vorherigen Ansprüche, **dadurch gekennzeichnet**, daß eine Bewegung der Vorrichtung nur dann auslösbar ist, wenn neben dem Geschwindigkeitssteuersignal noch ein Enable-Signal vom Achsrechner (**18**) an der Leistungsendstufe (**22**) anliegt.

10. Vorrichtung nach einem der vorherigen Ansprüche, **dadurch gekennzeichnet**, daß das Lightpen-Meßsystem (**8**) einen Zähler (**40**) für die Erfassung der Zeilenzahl und einen Zähler (**41**) für die Erfassung der Zeilenlänge aufweist.

11. Vorrichtung nach einem der vorherigen Ansprüche, **dadurch gekennzeichnet**, daß zur Konkrementenpositionseingabe ein Tracking-Ball (**46**) mit Meßsystem (**8**) und ein Fadenkreuz (**52**) vorgesehen ist.

12. Vorrichtung nach einem der vorherigen Ansprüche, **dadurch gekennzeichnet**, daß zur Konkrementenpositionseingabe ein Joystick (**50**) mit Meßsystem (**8**) und ein Fadenkreuz (**52**) vorgesehen ist.

13. Vorrichtung nach einem der vorherigen Ansprüche, **dadurch gekennzeichnet**, daß zur Konkrementenpositionseingabe eine Mouse (**48**) mit Meßsystem (**8**) und ein Fadenkreuz (**52**) vorgesehen ist.

14. Vorrichtung nach einem der vorherigen Ansprüche, **dadurch gekennzeichnet**, daß zur Konkrementenpositionseingabe eine dem Röntgenmonitor (**10, 12**) vorgesetzte Touch-Screen vorgesehen ist.

15. Vorrichtung nach einem der vorherigen Ansprüche, **dadurch gekennzeichnet**, daß zwischen Leitrechner (**2**) und Ortungssystem (**8**, **14**) galvanische Trennung besteht.

## Claims

1. Locating and positioning device in an apparatus for contact-free reduction of concrements in bodies of living beings, comprising a water cushion by means of which shockwaves are introduced into the body, computer-supported position-coordinates evaluation of the concrements and computer-controlled positioning of a patient's berth, **characterised by**
- computer-controlled slaving of the level of the water cushion and
- computer-controlled positioning of a located concrement (32) in the second focus of a ellipsoid of rotation on axes in the
x-direction (towards the foot/head),
y-direction (left/right),
z-direction (up/down), and
w-direction (pivoting around the longitudinal axis).

2. Device according to claim 1, **characterised in that** at least one, preferably two, X-ray or ultra-sound locating systems (10̸, 12, 14) are provided.

3. Device according to at least one of the above claims, **characterised in that** the position coordinates are received by an image-processing system (2, 8, 10̸, 12, 14).

4. Device according to at least one of the above claims, **characterised in that** the image-processing system comprises two X-ray recording apparatus (14) the central beams of which intersect, two thereto coupled and associated monitors (10̸, 12), a light-pen measuring system (8) which is connected to the monitors (10̸, 12) and the X-ray recording apparatus (14), and a master computer (2) which is connected to the light-pen measuring system (8).

5. Device according to one of the above claims, **characterised in that** each axis comprises a separate axis computer (18), an output end stage (22), a motor (24) with speed and angle transmitter (26) flanged thereto, an absolute position transmitter (28) and a mechanical end switch (30̸).

6. Device according to one of the above claims, **characterised in that** all movements of the positioning device are also controllable via a manual control (6).

7. Device according to one of the above claims, **characterised in that** the positioning device moves only as long as the release key is depressed (canopy-switch principle).

8. Device according to one of the above claims, **characterised in that** transgression of the movement range is prevented both by operational software limiters and operational hardware limiters (30̸).

9. Device according to one of the above claims, **characterised in that** a movement of the device can only be triggered if the output end stage (22) is in addition to to speed-control signal fed an enable signal by the axis computer (18).

10. Device according to one of the above claims, **characterised in that** the light-pen measuring system (8) comprises a counter (40̸) for acquiring the line number and a counter (41) for acquiring the line length.

11. Device according to one of the above claims, **characterised in that** a tracking ball (46) with measuring system (8) and a graticule (52) are provided for entering the concrement position.

12. Device according to one of the above claims, **characterised in that** a joystick (50̸) with measuring system (8) and graticule (52) are provided for entering the concrement position.

13. Device according to one of the above claims, **characterised in that** a mouse (48) with measuring system (8) and graticule (52) is provided for entering the concrement position.

14. Device according to one of the above claims, **characterised in that** a touch-screen in front of the X-ray monitor (10̸, 12) is provided for entering the concrement position.

15. Device according to one of the above claims, **characterised by** a galvanic separation between the master computer (2) and the locating system (8, 14).

## Revendications

1. Dispositif de localisation et de positionnement pour un dispositif permettant de fragmenter sans contact des concrétions dans le corps d'êtres vivants, comportant un coussin de liquide de couplage par l'intermédiaire duquel on introduit des ondes de choc dans le corps, avec un calcul assisté par ordinateur des coordonnées des concrétions et avec un positionnement commandé par ordinateur d'une table recevant le malade, caractérisé par
- une adaptation commandée par ordinateur du niveau du coussin de liquide de couplage et
- le positionnement commandé par ordinateur d'une concrétion (32) localisée, dans le second foyer d'une ellipsoïde de rotation, sur des axes dans
la direction x (vers les pieds/vers la tête)
la direction y (gauche/droite)
la direction z (vers le haut/vers le bas)
la direction w (pivotement autour de l'axe longitudinal)

2. Dispositif selon la revendication 1,, caractérisé par le fait qu'il est prévu au moins un, de préférence deux systèmes de localisation (10, 12, 14) par rayons X ou par ultrasons.

3. Dispositif selon l'une au moins des revendications précédentes, caractérisé par le fait que les coordonnées de position sont enregistrées par un dispositif de traitement d'image (2, 8, 10, 12, 14).

4. Dispositif selon l'une au moins des revendications précédentes, caractérisé par le fait que le dispositif de traitement d'image est constitué de deux appareils de radiographie (14) dont les rayons médians sont sécants, de deux moniteurs (10, 12) correspondants couplés à ceux-ci, d'un système de mesure à crayon lumineux (8) qui est connecté aux moniteurs (10, 12) et aux appareils de radiographie (14) et d'un calculateur de pilotage (2) connecté au système de mesure à crayon lumineux (8).

5. Dispositif selon l'une des revendications précédentes, caractérisé par le fait que chaque axe dispose d'un calculateur d'axe (18) individuel, d'un étage final de puissance (22), d'un moteur (24) avec un capteur de vitesse et d'angle (26) couplé, d'un capteur de position (28) absolu et d'un contacteur mécanique de fin de course (30).

6. Dispositif selon l'une des revendications précédentes, caractérisé par le fait que tous les déplacements du dispositif de positionnement peuvent également être commandés par une commande manuelle (6).

7. Dispositif selon l'une des revendications précédentes, caractérisé par le fait que le déplacement du dispositif de positionnement nest obtenu que dans la mesure où une touche d'actionnement est enfoncée (principe de l'homme mort).

8. Dispositif selon l'une des revendications précédentes, caractérisé par le fait que des limiteurs de déplacement logiciels et matériels (30) empêchent le dépassement des limites de déplacement.

9. Dispositif selon l'une des revendications précédentes, caractérisé par le fait qu'un déplacement du dispositif ne peut être déclenché que si l'étage final de puissance reçoit, en plus du signal de contrôle de vitesse, un signal de validation en provenance du calculateur d'axe (18).

10. Dispositif selon l'une des revendications précédentes, caractérisé par le fait que le système de mesure à crayon lumineux (8) présente un compteur (40) pour le comptage du nombre de lignes et un compteur (41) pour la mesure de la longueur des lignes.

11. Dispositif selon l'une des revendications précédentes, caractérisé par le fait qu'il est prévu pour l'entrée de la position de la concrétion une boule roulante (46) à système de mesure (8) et un réticule (52).

12. Dispositif selon l'une des revendications précédentes, caractérisé par le fait qu'il est prévu, pour l'entrée de la position de la concrétion, une manette (50) avec système de mesure (8) et un réticule (52).

13. Dispositif selon l'une des revendications précédentes, caractérisé par le fait qu'il est prévu, pour l'entrée de la position de la concrétion une souris (48) avec système de mesure (8) et un réticule (52).

14. Dispositif selon l'une des revendications précédentes, caractérisé par le fait qu'il est prévu, pour l'entrée de la position de la concrétion, un écran tactile placé devant le moniteur de radiographie (10, 12).

15. Dispositif selon l'une des revendications précédentes, caractérisé par le fait qu'il existe une séparation électrique entre le calculateur de pilotage (2) et le dispositif de localisation (8, 14).
